# EUROPÄISCHE PATENTSCHRIFT

(11) **EP 1 165 021 B1**
(45) Veröffentlichungstag und Bekanntmachung des Hinweises auf die Patenterteilung: **08.11.2006**
(21) Anmeldenummer: 00912632.7
(22) Anmeldetag: 22.03.2000
(51) Int. Cl.: A61Q 5/10, A61Q 5/12, A61K 8/55

(54) **PHOSPHATTYP TENSIDE KOMBINIERT MIT HAARKONDITIONERMITTELN IN HAARFÄRBUNGZUSAMMENSETZUNGEN**
PHOSPHATE-TYPE TENSIDES COMBINED WITH HAIR CONDITIONING AGENTS IN HAIR COLOURING COMPOSITIONS
TENSIOACTIFS DU TYPE PHOSPHATE COMBINES AVEC DES AGENTS CONDITIONNEURS POUR CHEVEUX DANS DES COMPOSITIONS DE COLORATION POUR CHEVEUX

(30) Priorität: 01.04.1999 DE 19914927
(43) Veröffentlichungstag der Anmeldung: 02.01.2002
(73) Patentinhaber: Henkel Kommanditgesellschaft auf Aktien, 40589 Düsseldorf (DE)
(72) Erfinder: AKRAM, Mustafa, D-22457 Hamburg (DE); WOLFF, Wolfgang, D-22941 Bargteheide (DE); ROHWEDER, Sandra, D-22763 Hamburg (DE)
(86) Internationale Anmeldenummer: PCT/EP2000/002538
(87) Internationale Veröffentlichungsnummer: WO 2000/059457

(56) Entgegenhaltungen:
- EP-A- 0 013 713
- EP-A- 0 566 049
- EP-A- 0 872 229
- WO-A-92/18093
- WO-A-97/14406
- WO-A-98/56333
- DE-A- 4 408 506

## Beschreibung

Die vorliegende Erfindung betrifft eine pflegende Wirkstoffkombination zur Behandlung keratinischer Fasern, insbesondere menschlicher Haare, Haarfärbemittel, die diese enthalten, sowie die Verwendung dieser Wirkstoffkombination in Haarfärbemitteln.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen.

So werden beispielsweise übliche Haarfärbemittel auf Basis von Oxidationsfarbstoffen formuliert. Häufig werden auch Kombinationen von Oxidationsfarbstoffen und direktziehenden Farbstoffen zur Erzielung spezieller Nuancen eingesetzt. Färbemittel auf Basis von Oxidationsfarbstoffen führen zu brillanten und dauerhaften Farbtönen. Sie bedingen allerdings den Einsatz starker Oxidationsmittel wie beispielsweise Wasserstoffperoxid-Lösungen. Dies kann das zu färbende Haar schädigen. Diesen Schädigungen muß dann mit entsprechenden Pflegeprodukten entgegengewirkt werden.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen.

Es besteht daher weiterhin ein Bedarf an Wirkstoffen bzw. Wirkstoffkombinationen mit guten pflegenden Eigenschaften und guter biologischer Abbaubarkeit, bei denen unerwünschte Kumulationen auf dem Haar ausgeschlossen sind.

Es wurde nun überraschenderweise gefunden, daß eine Kombination aus bestimmten kationischen Tensiden mit weiteren konditionierenden Substanzen die oben erwähnten Nachteile nicht aufweisen und gleichzeitig den Griff, die Naßkämmbarkeit sowie den Glanz der behandelten Haare verbessern.

Ein erster Gegenstand der Erfindung sind somit Mittel zur pflegenden Behandlung keratinischer Fasern, insbesondere menschlicher Haare, die mindestens ein Tensid der Formel (I) 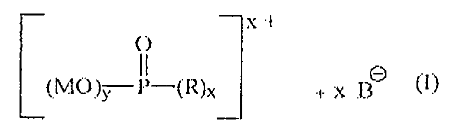 enthalten sowie mindestens eine konditionierende Komponente und mindestens einen Farbstoff und/oder mindestens ein Farbstoffvorprodukt.

In der Formel (I) steht y für eine ganze Zahl von 0 bis 2 x für eine ganze Zahl von 1 bis 3 mit der Maßgabe, daß die Summe aus x und y gleich 3 ist.

In den erfindungsgemäß einzusetzenden Tensiden steht ferner M für Wasserstoff, ein Äquivalent eines Alkali- oder Erdalkalimetallkations, ein Ammoniumkation oder einen Alkylrest mit. 1 bis 4 Kohlenstoffatomen, der gegebenenfalls mit einer oder mehreren Hydroxygruppe(n) substituiert ist. Besonders bevorzugt sind Verbindungen, bei denen M für ein Natriumkation steht.

Weiterhin steht B in der Formel (I) der erfindungsgemäß einzusetzenden Tenside für ein Äquivalent eines physiologisch verträglichen Anions. Als Anion eignen sich z.B. Chlorid, Bromid, Jodid, Sulfat, Perchlorat, Tetrafluorborat, Tetraphenylborat und Tetrachlorozinkat. Bevorzugt ist das Chloridion.

R steht in den erfindungsgemäßen Tensiden der Formel (I) für einen Rest der Formel (II), 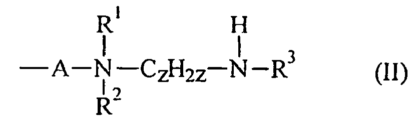 in der z für eine ganze Zahl von 1 bis 4, insbesondere für 3, steht und R¹ und R² unabhängig voneinander für einen C₁- bis C₄-Alkylrest stehen, der gegebenenfalls mit einer oder mehreren Hydroxygruppe(n) oder einer Acylgruppe substituiert ist.

A steht erfindungsgemäß für eine der Einheiten -O-CH₂-CH₂-CH₂-, -O-CH₂-CH₂- oder -O-CH₂-CHOH-CH₂-, wobei die Einheit -O-CH₂-CHOH-CH₂- besonders bevorzugt ist.

Der Rest R³ steht für
(a) einen verzweigten oder unverzweigten, gesättigten C₈- bis C₁₈-Acylrest oder
(b) einen verzweigten oder unverzweigten, einfach oder mehrfach ungesättigten C₈- bis C₁₈-Acylrest.

Besonders bevorzugte gesättigte Reste R³ sind der Rest der Stearinsäure sowie die Reste der Mischung der Fettsäuren, die man aus Kokosöl gewinnt.

Ein besonders bevorzugter ungesättigter Rest R³ ist der Rest der Linolsäure. Überraschenderweise wurde gefunden, daß sich Verbindungen der Formel (1), bei denen R³ der Rest der Linolsäure ist, durch eine höhere Verträglichkeit mit dem Emulgatorsystem auszeichnen. Dies bedeutet, daß sich diese Substanzen leichter in die Formulierungen einarbeiten lassen. Weiterhin weisen Formulierungen mit Verbindungen der Formel (I), bei denen R³ für den Rest der Linolsäure steht, einen deutlich höheren Pflegeeffekt im Vergleich zu Verbindungen mit gesättigten Fettsäureresten auf.

Beispiele für die als Substituenten in den erfindungsgemäßen Verbindungen genannten C₁- bis C₄-Alkylgruppen sind die Gruppen Methyl, Ethyl, Propyl, Isopropyl und Butyl. Ethyl- und Methylgruppen sind bevorzugte Alkylgruppen. Ganz besonders bevorzugt sind Methylgruppen.

Verbindungen der Formel (I) sind bereits bekannt. So werden in der EP-A1-13 713 die tensidischen Eigenschaften dieser Verbindungen allgemein beschrieben. Ferner ist aus der DE-A1-44 08 506 der Einsatz einer Verbindung der Formel (I) in Haarfärbemitteln bereits bekannt. Diesen Schriften sind aber keine Hinweise auf die synergistische Steigerung der Pflegewirkung der erfindungsgemäßen Wirkstoffkombinationen zu entnehmen.

Ganz besonders bevorzugte Verbindungen der Formel (I) sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Erfindungsgemäß werden die Verbindungen der Formel (I) in Mengen von 0,1 bis 5 Gew.-%, insbesondere in Mengen von 0,2 bis 2 Gew.-%, jeweils bezogen auf das gesamte Mittel, in den beanspruchten Mitteln eingesetzt.

Bevorzugte konditionierende Wirkstoffe sind erfindungsgemäß die niedermolekularen, quartären Ammoniumverbindungen. Besonders bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Ganz besonders bevorzugte Verbindungen sind die Halogenide des Cetyltrimethylammoniumkations, insbesondere das Bromid.

Zu den bevorzugten quartären Ammoniumverbindungen zählen ferner die quartären Esterverbindungen, sogenannte "Esterquats", wie die unter dem Warenzeichen Stepantex^{®} vertriebenen Methyl-hydroxyalkyl-dialkoyloxyalkyl-ammonium-methosulfate sowie die unter dem Warenzeichen Dehyquart^{®} vertriebenen Produkte. Ganz besonders bevorzugt ist die unter dem Handelsnamen Dehyquart^{®} F75 vertriebene Mischung von Fettalkoholen mit Methyltriethanolammoniummethylsulfatdialkylestem.

Eine weitere bevorzugte Gruppe quartärer Ammoniumverbindungen sind die quaternisierten Derivate des Imidazolins, wie beispielsweise das unter dem Markennamen Rewoquat^{®} W 75 PG (INCI-Bezeichnung: Quaternium-27) vertriebene Produkt.

Als konditionierende Wirkstoffe bevorzugt sein können weiterhin kationische Polymere. Dies sind Polymere, die üblicherweise ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 (INCI-Bezeichnung: Polyquatemium-10) sind bevorzugte quaternierte Cellulose-Derivate.
- polymere Dimethyldiallylammoniumsalze und deren Copolymere mit Estern und Amiden von Acrylsäure und Methacrylsäure sowie den freien Säuren. Die unter den Bezeichnungen Merquat^{®}100 (Poly(dimethyldiallylammoniumchlorid)), Merquat^{®} 280 (Dimethyldiallylammoniumchlorid-Acrylsäure-Copolymer), Merquat^{®}550 (Dimethyldiallylammoniumchlorid-Acrylamid-Copolymer) sowie Merquat^{®} Plus 3300 (Dimethyldiallylammoniumchlorid-Acrylamid-Acrylsäure-Terpolymer) im Handel erhältlichen Produkte sind Beispiele für solche kationischen Polymere.
- Copolymere des Vinylpyrrolidons mit quatemierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich. Ein weiteres Beispiel für ein derartiges Copolymer ist das unter dem Handelsnamen Gafquat^{®} HS 100 vertriebene Copolymer aus Vinylpyrrolidon und Methacrylamidopropyltrimethylammoniumchlorid.
- Vinylpyrrolidon-Methoimidazoliniumchlorid-Copolymere, wie sie unter der Bezeichnung Luviquat^{®} angeboten werden.
- quaternierter Polyvinylalkohol
- Polyquatemium-37, wie es unter dem Handelsnamen Salcare^{®} SC96 vertrieben wird,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Bevorzugt sind kationische Polymere der vier erstgenannten Gruppen, sowie insbesondere die Polymeren, die unter dem Handelsnamen Mirapol^{®} A15 (INCI-Bezeichung: Polyquaternium-2) beziehungsweise Gafquat^{®} 755N (INCI-Bezeichnung: Polyquaternium-11) vertrieben werden. Polyquaternium-2 ist in Verbindung mit den Tensiden der Formel (I) eine ganz besonders bevorzugte konditionierende Komponente.

Insbesondere bei der Kombination der Verbindungen der Formel (I) mit kationischen Polymeren als konditionierende Komponenten wurden überraschend starke synergistische Wirkungen der Komponenten hinsichtlich des gesamten Pflegeeffektes beobachtet.

Eine weitere Gruppe der konditionierenden Komponenten bilden die Proteinderivate. Die Proteinderivate können auf tierischen oder pflanzlichen Proteinen basieren. Geeignete Ausgangsstoffe sind beispielsweise Keratin, Kollagen, Elastin, Weizenproteine, Milchproteine, Eiweißproteine, Seidenproteine, Mandelproteine, Sojaproteine sowie Proteine aus Tierhäuten.

Entsprechende Proteinhydrolysate werden jeweils als Produktgemisch aus dem sauer, basisch und/oder enzymatisch katalysierten Abbau dieser Proteine erhalten. Ein Beispiel eines erfindungsgemäß bevorzugten Proteinhydrolysats ist das unter dem Handelsnamen Crotein^{®} C vertriebene Collagenderivat (INCI-Bezeichnung: hydrolized collagen).

Kationische Derivate erhält man durch anschließende Umsetzung mit Verbindungen, die üblicherweise quartäre Ammoniumgruppen tragen oder durch Umsetzung mit entsprechenden Aminen und anschließender Quaternierung. Eine Reihe solcher quartärer Proteinhydrolysate sind als Handelsprodukte erhältlich, beispielsweise unter den Handelsnamen Lamequat^{®} L (kationisches Kollagenhydrolysat; INCI-Bezeichnung: Lauryldimonium Hydroxypropylamino Hydrolyzed Animal Protein; Henkel), Croquat^{®} WKP (tierisches Keratinhydrolysat; INCI-Bezeichnung: Aqua, Cocodimonium Hydroxypropyl Hydrolyzed Keratin; Croda), Hydrotriticum^{®} QL (kationisches Weizenproteinhydrolysat; INCI-Bezeichnung: Lauryldimonium Hydroxypropyl Hydrolyzed Wheat Protein; Croda) sowie Crotein^{®} Q (kationisches Kollagenhydrolysat; INCI-Bezeichnung: Hydroxypropyltrimonium Hydrolyzed Collagen; Croda).

In einer ersten bevorzugten Ausführungsform werden Proteinderivate tierischen Ursprungs bevorzugt. Besonders bevorzugt sind die Proteinhydrolysate des tierischen Keratins. Da die Zusammensetzung hinsichtlich der vorhandenen Aminosäuresequenzen der des Humanhaares sehr ähnlich ist, resultiert eine hohe Affinität derartiger Produkte zum menschlichen Haar. Beispiele hierfür sind die unter den Handelsnamen Nutrilan^{®} Keratin W und Promois^{®} WK vertriebenen Produkte.

Es kann aber auch gemäß einer weiteren Ausführungsform der vorliegenden Erfindung bevorzugt sein, Proteinderivate pflanzlichen Ursprungs einzusetzen. Ein bevorzugtes pflanzliches Proteinhydrolysat ist das unter dem Handelsnamen Gluadin^{®} WQ vertriebene quaternäre Weizenproteinhydrolysat.

Ferner sind quaternisierte Galactomannan-Polysaccharide bevorzugte Konditioniermittel. Erfindungsgemäß bevorzugte Galactomannan-Polysaccharide sind die quaternären Guar Gum-Derivate, insbesondere quaternäre Hydroxy-C₂-C₄-alkyl-Guar Gums, d.h. der quaternäre Propylenglykolether des Guar Gums, insbesondere das Hydroxypropyl-Guarhydroxypropyltrimoniumchlorid. Weitere geeignete Derivate sind beispielsweise das quaternäre Hydroxyethyl-Guar und das quaternäre Hydroxybutyl-Guar. Geeignete Handelsprodukte sind beispielsweise unter den Markennamen Jaguar^{®} C-17 und Jaguar^{®} C-162 auf dem Markt. Eine weitere Gruppe von geeigneten Galactomannanen sind die aus den Früchten des Johannisbrotbaums gewonnenen quaternären Polysaccharide.

In einer weiteren Ausführungsform der vorliegenden Verbindung sind die konditionierenden Komponenten ausgewählt aus den Silikonölen. Als Silikonöle können beispielsweise folgende Verbindungen eingesetzt werden:
- Oligomere Polydimethylcyclosiloxane (INCI-Bezeichnung: Cyclomethicone), insbesondere die tetramere und pentamere Verbindung,
- Hexamethyl-Disiloxan,
- Polyphenylmethylsiloxane (INCI-Bezeichnung: Phenyl Trimethicone),
- Dimethylsiloxane-Dimethylpolysiloxanol-Gemische (INCI-Bezeichnung: Cyclomethicone (and) Dimethiconol),
- Silicon-Glykol-Copolymere (INCI-Bezeichnung: Dimethicone Copolyol),
- aminofunktionelle Polydimethylsiloxane und
- hydroxylaminomodifzierte Silicone.

Solche Verbindungen sind auf dem Markt erhältlich. Bekannte Handelsprodukte sind beispielsweise DC^{®}344 Fluid, DC^{®}345 Fluid, DC^{®}200 Fluid, DC^{®}556, DC^{®}190, DC^{®}193 SU und DC^{®}Q2-1401 des Herstellers Dow Coming sowie die Produkte Abil^{®}K4, Abil^{®}K520 und Abil^{®}B8839 des Herstellers Th. Goldschmidt AG.

Besonders bevorzugt sind die Dimethylsiloxane-Dimethylpolysiloxanol-Gemische sowie die aminogruppenhaltigen Silikonöle, wie beispielsweise die im Handel erhältlichen Produkte Q2-7224 (Hersteller: Dow Corning; ein stabilisiertes Trimethylsilylamodimethicon), Dow Coming 939 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80). Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Die konditionierenden Komponenten sind bevorzugt in Mengen von 0,05 bis 5 Gew.-%, insbesondere von 0,1 bis 2 Gew.-%, jeweils bezogen auf das gesamte Mittel, in den erfindungsgemäßen Mitteln enthalten. Bei Silikonölen können Mengen von 0,05 bis 10 Gew.-%, insbesondere von 0,2 bis 5 Gew.-%, ganz besonders Mengen von 0,5 bis 2 Gew.-%, jeweils bezogen auf das gesamte Mittel, in den erfindungsgemäßen Mitteln bevorzugt sein.

Die Art des erfindungsgemäß verwendeten Haarbehandlungsmittels unterliegt prinzipiell keinen Beschränkungen. Die erfindungsgemäßen Mittel können sowohl auf dem Haar verbleiben, als auch nach einer Einwirkzeit von wenigen Sekunden bis zu 45 Minuten wieder ausgespült werden. Beispiele für erfindungsgemäß verwendete Mittel sind Shampoos, Spülungen, Kuren, Konditioniermittel, Tönungsmittel, Färbemittel, Dauerwellmittel, Fixiermittel, Haarsprays und Fönwellen. Der Einsatz der erfindungsgemäßen Wirkstoffkombinationen in Rinse-off-Produkte kann eine bevorzugte Ausführungsform sein.

Ein zweiter Gegenstand der vorliegenden Erfindung sind Haarbehandlungsmittel, die die oben beschriebene Wirkstoffkombination sowie mindestens ein Farbstoffvorprodukt und/oder mindestens einen Farbstoff enthalten. Die mit den erfindungsgemäßen Mitteln erzielten Färbungen zeichnen sich durch ihre verbesserten Echtheitseigenschaften bei deutlich verbessertem Pflegezustand der Fasern aus.

In einer ersten Ausführungsform dieses Gegenstandes der vorliegenden Erfindung kann das Farbstoffvorprodukt ein Oxidationsfarbstoffvorprodukt vom Typ Entwickler sein. Es können auch mehrere Entwickler gemeinsam in den erfindungsgemäßen Mitteln eingesetzt werden.

Entwicklersubstanzen sind üblicherweise aromatische oder heterocyclische Ringsysteme, die durch zwei in ortho- oder para-Stellung zueinander stehende reaktive Gruppen, i.a. Hydroxy- oder Aminogruppen, gekennzeichnet sind. Solche Verbindungen sind beispielsweise primäre aromatische Amine mit einer weiteren, in para- oder ortho-Position befindlichen freien oder substituierten Hydroxy- oder Aminogruppe, ferner Diaminopyridinderivate, heterocyclische Hydrazonderivate oder 4-Aminopyrazolonderivate.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Amino-methyl-4-aminophenol, 2-Hydroxymethyl-4-aminophenol, Bis-(2-hydroxy-5-amino-phenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin sowie 4-Hydroxy-2,5,6-triaminopyrimidin.

Zur Nuancierung der erzielbaren Farbtöne können die erfindungsgemäßen Mittel weiterhin noch eine oder mehrere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 3-Amino-6-methoxy-2-methylaminophenol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)-benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-3,4-diaminopyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-3-aminophenol, 2-Methylresorcin, 5-Methylresorcin, 2,5-Dimethylresorcin, 2-Amino-3-hydroxypyridin sowie 2,6-Dihydroxy-3,4-diaminopyridin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer zweiten bevorzugten Ausführungsform dieses Gegenstandes der vorliegenden Erfindung kann das Farbstoffvorprodukt ein Derivat des Indolins der Formel (IIIa) sein, 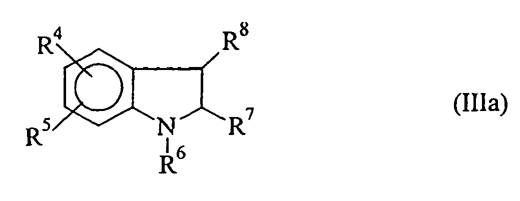 in der unabhängig voneinander R⁶ steht für Wasserstoff, eine C₁- bis C₄-Alkylgruppe oder eine C₁- bis C₄-Hydroxy-alkylgruppe, R⁷ steht für Wasserstoff oder eine -COOH-Gruppe, wobei die -COOH-Gruppe auch in Salzform mit einem physiologisch verträglichen Kation vorliegen kann, R⁸ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁹, in der R⁹ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

In einer dritten bevorzugten Ausführungsform dieses Gegenstandes der vorliegenden Erfindung kann das Farbstoffvorprodukt ein Derivat des Indols der Formel (IIIb) sein, 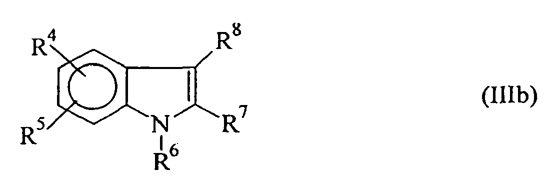 in der unabhängig voneinander R⁶ steht für Wasserstoff, eine C₁- bis C₄-Alkylgruppe oder eine C₁- bis C₄-Hydroxy-alkylgruppe, R⁷ steht für Wasserstoff oder eine -COOH-Gruppe, wobei die -COOH-Gruppe auch in Salzform mit einem physiologisch verträglichen Kation vorliegen kann, R⁸ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁹, in der R⁹ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

Bevorzugte Stoffe der Formel (IIIa) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (IIIb) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Aminoindol und 4-Aminoindol.

Ganz besonders bevorzugt sind 5,6-Dihydroxyindol sowie 5,6-Dihydroxyindolin.

In einer ersten bevorzugten Variante der oben beschriebenen Ausführungsformen werden die Mittel derart formuliert, daß sie als Farbstoffvorprodukte nur Indol- und/oder Indolinderivate der Formeln (IIIa) und (IIIb) enthalten und frei sind von üblichen Oxidationsfarbstoffvorprodukten vom Entwickler- bzw. Kupplertyp.

In einer zweiten bevorzugten Variante der oben beschriebenen Ausführungsformen können die erfindungsgemäßen Mittel neben den Indol- und/oder Indolinderivaten der Formeln (IIIa) und (IIIb) auch noch übliche Oxidationsfarbstoffvorprodukte vom Entwickler- bzw. Kupplertyp enthalten.

Es kann erfindungsgemäß besonders bevorzugt sein, die Indol- und/oder die Indolinderivate der Formeln (IIIa) und (IIIb) in Kombination mit einer oder mehreren Kupplerkomponenten in Haarfärbemitteln einzusetzen. Beispielhaft sei an dieser Stelle ausdrücklich auf die oben genannten Kupplerkomponenten verwiesen.

Weiterhin kann es erfindungsgemäß bevorzugt sein, die Indol- und/oder Indolinderivate der Formel (IIIa) und (IIIb) in Kombination mit mindestens einer Aminosäure oder einem Oligopeptid in Haarfärbemitteln einzusetzen. Es kann erfindungsgemäß weiterhin bevorzugt sein, wenn die Aminosäure eine α-Aminosäure ist. Ganz besonders bevorzugte α-Aminosäuren sind Arginin, Ornithin, Lysin und Histidin.

In einer weiteren bevorzugten Ausführungsform enthalten die erfindungsgemäßen Haarbehandlungsmittel direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Indigo, Sedre und Alkannawurzel enthalten.

Es ist nicht erforderlich, daß die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbemitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher industrie- und Handelsunternehmen für Arzneimittel, Reformwaren und Körperpflegemittel e.V., Mannheim, Bezug genommen.

Färbungen von besonderer Farbtiefe können erreicht werden, wenn die Mittel neben den Farbstoffen und/oder Farbstoffvorprodukten zusätzlich noch ein Öl des Wiesenschaumkrauts (INCI-Bezeichnung: Meadowfoam Seed Oil) enthalten.

In einer ganz besonders bevorzugten Ausführungsform der vorliegenden Erfindung enthalten die Mittel mindestens einen Farbstoff und/oder ein Farbstoffvorprodukt, Polyquaternium-2 sowie die unter der INCI-Bezeichnung Linoleamidopropyl PG-Dimonium Chloride Phosphate bekannte Verbindung der Formel (I).

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Flüssigkeiten, Gele oder auch tensidhaltige schäumende Lösungen. z.B. Shampoos, Schaumaerosole oder andere Zubereitungen, die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Haarbehandlungsmittel können weiterhin alle für solche Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Überraschenderweise wurde gefunden, daß anionische Tenside in die erfindungsgemäßen Mittel eingearbeitet werden können, ohne daß sich nennenswerte Niederschläge mit den kationischen Komponenten bilden.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolycthylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Alkylamidoamine, insbesondere Fettsäureamidoamine, wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Schließlich enthalten die erfindungsgemäßen Mittel bevorzugt noch einen Fettstoff.

Bevorzugte Fettstoffe sind lineare und verzweigte, gesättigte und ungesättigte Fettalkohole oder natürliche Fettalkoholgemische mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Haarbehandlungsmittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Mittels eingesetzt.

Ein weiterer Gegenstand der vorliegenden Erfindung ist die Verwendung der Mittel nach einem der Ansprüche 3 bis 12 zum Färben keratinischer Fasern.

Die nachfolgenden Beispiele sollen den Gegenstand der vorliegenden Erfindung näher erläutern.

Alle Mengenangaben in den Beispielen sind Gewichtsteile.

### Beispiel 1

| | |
|---|---|
| Ammoniumcarbopollösung, 1%ig in Wasser¹ | 17,25 |
| Ammoniumrohagitlösung, 6%ig in Wasser² | 5,25 |
| Oleth-7³ | 5,70 |
| Kaliumoleinseife, 12,5%ig in Wasser | 12,75 |
| Kaliumricinusseife, 12,5%ig in Wasser | 3,45 |
| Plantaren^{®} 2000⁴ | 0,53 |
| Titandioxid Anatas, Typ AS 05 | 0,48 |
| Cetiol^{®} V⁵ | 3,45 |
| Cetylalkohol | 16,80 |
| Glycerinmonostearat NSE⁶ | 2.85 |
| Phospholipid EFA⁷ | 0,85 |
| Tetrasodium EDTA | 0,46 |
| Kieselsäure, hochdispers, pyrogen | 0,11 |
| p-Toluylendiamin | 2,41 |
| Resorcin | 0,86 |
| 3-Aminophenol | 0,26 |
| 1-Methoxy-2-amino-4-(2-hydroxyethylamino)-benzol | 0,11 |
| 1,2-Propylenglykol USP | 1,05 |
| Methoxybutanol | 1,43 |
| Ammoniak, 25%ig in Wasser | ad pH 9,0 |
| Ascorbinsäure | 0,06 |
| Mirapol^{®} A 15⁸ | 0,19 |
| Parfüm | 0,43 |
| Wasser | ad 100,00 |

| | |
|---|---|
| ¹ Lösung eines Ammoniumsalzes eines Methacrylsäure-Methylacrylat-Copolymeren (INCI-Bezeichnung: Ammonium Polyacrylate) (Röhm GmbH) ² Lösung eines Ammoniumsalzes eines Acrylsäure-Polymeren (INCI-Bezeichnung: Ammonium Acrylates Copolymer) (Goodrich) ³ Oleylalkohol mit 7 EO-Einheiten (Henkel) ⁴ C₈₋₁₆-Alkyl-1,4-Polyglucosid (ca. 51% Aktivsubstanz; INCI-Bezeichnung: Decyl Glucoside) (Henkel) ⁵ Ölsäuredecylester (INCI-Bezeichnung: Decyl Oleate) (Henkel) ⁶ (INCI-Bezeichnung: Glyceryl Strearate) (Oleofina) ⁷ Verbindung der Formel (I) (ca. 30% Aktivsubstanz; INCI-Bezeichnung: Linoleamidopropyl PG-Dimonium Chloride Phosphate) (Mona) ⁸ ca. 64% Aktivsubstanz; INCI-Bezeichnung: Polyquaternium-2 (Rhodia) | |

Dieses Mittel wurde mit einer wäßrigen, 6%igen Wasserstoffperoxidlösung im Verhältnis 1:1 vermischt und auf eine hellbraune, zu 80% ergraute Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war dunkelbraun eingefärbt und wies eine sehr gute Grauabdeckung auf.

### Beispiel 2

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid EFA | 0,85 |
| Tetrasodium EDTA | 0,46 |
| Kieselsäure, hochdispers, pyrogen | 0,11 |
| p-Toluylendiamin | 2,12 |
| Resorcin | 0,63 |
| 3-Aminophenol | 0,20 |
| 1-Methoxy-2-amino-4-(2-hydroxyethylamino)-benzol | 0,05 |
| 1,2-Propylenglykol USP | 1,05 |
| Methoxybutanol | 1,43 |
| Ammoniak, 25%ig in Wasser | ad pH 10,0 |
| Ascorbinsäure | 0,06 |
| Cetyltrimethylammoniumbromid | 0,50 |
| Parfüm | 0,43 |
| Wasser | ad 100,00 |

Dieses Mittel wurde mit einer wäßrigen, 6%igen Wasserstoffperoxidlösung im Verhältnis 1:1 vermischt und auf eine dunkelblonde, zu 50% ergraute Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war hellbraun eingefärbt und wies eine sehr gute Grauabdeckung auf.

### Beispiel 3

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid EFA | 0,85 |
| Tetrasodium EDTA | 0,46 |
| Kieselsäure, hochdispers, pyrogen | 0,11 |
| p-Toluylendiamin | 0,84 |
| Resorcin | 0,21 |
| 3-Aminophenol | 0,05 |
| 4-Chlorresorcin | 0,15 |
| 1,2-Propylenglykol USP | 1,05 |
| Methoxybutanol | 1,43 |
| Ammoniak, 25%ig in Wasser | ad pH 10,5 |
| Ascorbinsäure | 0,06 |
| Rewoquat^{®} W 75 PG⁹ | 0,30 |
| Parfüm | 0,43 |
| Wasser | ad 100,00 |

| | |
|---|---|
| ⁹ 1-Methyl-2-nortalgalkyl-3-Talgfettsäureamidocthylimidazoliniummethosulfat (ca. 75% Aktivsubstanz in Propylenglykol; INCI-Bezeichnung: Quaternium 27) (Witco Surfactants GmbH) | |

Dieses Mittel wurde mit einer wäßrigen, 6%igen Wasserstoffperoxidlösung im Verhältnis 1:1 vermischt und auf eine mittelblonde, zu 50% ergraute Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war dunkelblond eingefärbt und wies eine sehr gute Grauabdeckung auf.

### Beispiel 4

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid EFA | 0,85 |
| Tetrasodium EDTA | 0,46 |
| Kieselsäure, hochdispers, pyrogen | 0,22 |
| p-Toluylendiamin | 1,33 |
| Resorcin | 0,48 |
| 3-Aminophenol | 0,10 |
| 1-Methoxy-2-amino-4-(2-hydroxyethylamino)-benzol | 0,02 |
| 1,2-Propylenglykol USP | 1,05 |
| Methoxybutanol | 1,43 |
| Ammoniak, 25%ig in Wasser | ad pH 9,5 |
| Ascorbinsäure | 0,06 |
| Polymer JR^{®} 400¹⁰ | 1,00 |
| Parfüm | 0,43 |
| Wasser | ad 100,00 |

| | |
|---|---|
| ¹⁰ quaternierte Hydroxyethylcellulose (INCI-Bezeichnung: Polyquaternium-10) (Amerchol) | |

Dieses Mittel wurde mit einer wäßrigen, 1,5%igen Wasserstoffperoxidlösung im Verhältnis 1:2 vermischt und auf eine dunkelblonde Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war hellbraun eingefärbt.

### Beispiel 5

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid SV¹¹ | 0,85 |
| Tetrasodium EDTA | 0,46 |
| Kieselsäure, hochdispers, pyrogen | 0,11 |
| p-Toluylendiamin | 2,41 |
| Resorcin | 0,86 |
| 3-Aminophenol | 0,26 |
| 1-Methoxy-2-amino-4-(2-hydroxyethylamino)-benzol | 0,11 |
| 1,2-Propylenglykol USP | 1,05 |
| Methoxybutanol | 1,43 |
| Ammoniak, 25%ig in Wasser | ad pH 9,0 |
| Ascorbinsäure | 0,06 |
| Gafquat^{®} 755N¹² | 0,50 |
| Parfüm | 0,43 |
| Wasser | ad 100,00 |

| | |
|---|---|
| ¹¹ Verbindung der Formel (I) (ca. 41,5% Aktivsubstanz; INCI-Bezeichnung: Stearamidopropyl PG-Dimonium Chloride Phosphate (and) Cetyl Alcohol) (Mona) ¹² quatemisiertes Vinylpyrrolidon-Dimethylaminoethylmethacrylat-Copolymer-Diethylsulfat (ca. 19% Aktivsubstanz; INCI-Bezeichnung: Polyquatemium-11) (ISP) | |

Dieses Mittel wurde mit einer wäßrigen, 6%igen Wasserstoffperoxidlösung im Verhältnis 1:1 vermischt und auf eine hellbraune, zu 50% ergraute Normalhaarsträhne aufgetragen. Nach einer Einwirkzeit von 30min bei 25°C wurde die Strähne mit Wasser ausgespült, nachshampooniert und mit dem Fön getrocknet. Die so erhaltene Strähne war dunkelbraun eingefärbt und wies eine sehr gute Grauabdeckung auf.

### Beispiel 6

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid SV | 0,85 |
| Tetrasodium EDTA | 0,46 |
| Kieselsäure, hochdispers, pyrogen | 0,11 |
| p-Toluylendiamin | 2,12 |
| Resorcin | 0,63 |
| 3-Aminophenol | 0,20 |
| 1-Methoxy-2-amino-4-(2-hydroxyethylamino)-benzol | 0,05 |
| 1,2-Propylenglykol USP | 1,05 |
| Methoxybutanol | 1,43 |
| Ammoniak, 25%ig in Wasser | ad pH 8,9 |
| Ascorbinsäure | 0,06 |
| Crotein^{®} C¹³ | 0,30 |
| Parfüm | 0,43 |
| Wasser | ad 100,00 |

| | |
|---|---|
| ¹³ Gelatine-Hydrolysat (ca. 93% Aktivsubstanz; INCI-Bezeichnung: Hydrolyzed Collagen) (Croda) | |

Dieses Mittel wurde mit einer wäßrigen, 3%igen Wasserstoffperoxidlösung im Verhältnis 1:1 vermischt und auf eine mittelblonde Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war mittelbraun eingefärbt.

### Beispiel 7

| | |
|---|---|
| Ammoniumearbopollösung, 1 %ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid SV | 0,85 |
| Tetrasodium EDTA | 0,46 |
| Kieselsäure, hochdispers, pyrogen | 0,11 |
| p-Toluylendiamin | 0,84 |
| Resorcin | 0,21 |
| 3-Aminophenol | 0,05 |
| 4-Chlorresorcin | 0,16 |
| 1,2-Propylenglykol USP | 1,05 |
| Methoxybutanol | 1,43 |
| Ammoniak, 25%ig in Wasser | ad pH 9,0 |
| Ascorbinsäure | 0,06 |
| Gluadin^{®} WK¹⁴ | 0,70 |
| Parfüm | 0,43 |
| Wasser | ad 100,00 |

| | |
|---|---|
| ¹⁴ Weizenproteinhydrolysat-Fettsäure-Kondensat (ca. 30% Aktivsubstanz; INCI-Bezeichnung: Sodium Cocoyl Hydrolyzed Wheat Protein) (Henkel) | |

Dieses Mittel wurde mit einer wäßrigen, 3%igen Wasserstoffperoxidlösung im Verhältnis 1:2 vermischt und auf eine mittelblonde Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war dunkelblond eingefärbt.

### Beispiel 8

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid PTC¹⁵ | 0,85 |
| Tetrasodium EDTA | 0,46 |
| Kieselsäure, hochdispers, pyrogen | 0,11 |
| p-Toluylendiamin | 2,41 |
| Resorcin | 0,86 |
| 3-Aminophenol | 0,26 |
| 1-Methoxy-2-amino-4-(2-hydroxyethylamino)-benzol | 0,11 |
| 1,2-Propylenglykol USP | 1,05 |
| Methoxybutanol | 1,43 |
| Ammoniak, 25%ig in Wasser | ad pH 9,1 |
| Ascorbinsäure | 0,06 |
| Merquat^{®} 280¹⁶ | 0,20 |
| Parfüm | 0,43 |
| Wasser | ad 100,00 |

| | |
|---|---|
| ¹⁵ Verbindung der Formel (I) (ca. 47% Aktivsubstanz; INCI-Bezeichnung: Cocamidopropyl PG-Dimonium Chloride Phosphate) (Mona) ¹⁶ Dimethyldiallylammoniumchlorid-Acrylsäure-Copolymer (ca. 35% Aktivsubstanz; INCI-Bezeichnung: Polyquaternium-22) (Chemviron) | |

Dieses Mittel wurde mit einer wäßrigen, 6%igen Wasserstoffperoxidlösung im Verhältnis 1:1 vermischt und auf eine hellbraune, zu 50% ergraute Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war dunkelbraun eingefärbt und wies eine sehr gute Grauabdeckung auf.

### Beispiel 9

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid PTC | 0,85 |
| Tetrasodium EDTA | 0,46 |
| Kieselsäure, hochdispers, pyrogen | 0,11 |
| p-Toluylendiamin | 2,12 |
| Resorcin | 0,63 |
| 3-Aminophenol | 0,20 |
| 1-Methoxy-2-amino-4-(2-hydroxyethylamino)-benzol | 0,05 |
| 1,2-Propylenglykol USP | 1,05 |
| Methoxybutanol | 1,43 |
| Ammoniak, 25%ig in Wasser | ad pH 9,1 |
| Ascorbinsäure | 0,06 |
| Jaguar^{®} C-17¹⁷ | 0,30 |
| Parfüm | 0,43 |
| Wasser | ad 100.00 |

| | |
|---|---|
| ¹⁷ Guarhydroxypropyltrimethylammoniumchlorid (INCI-Bezeichnung: Guar Hydroxypropyltrimonium Chloride) (Rhodia Inc.) | |

Dieses Mittel wurde mit einer wäßrigen, 3%igen Wasserstoffperoxidlösung im Verhältnis 1:2 vermischt und auf eine hellbraune Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war mittelbraun eingefärbt.

### Beispiel 10

| | |
|---|---|
| Ammoniumcarbopollösung, 1%ig in Wasser | 17,25 |
| Ammoniumrohagitlösung, 6%ig in Wasser | 5,25 |
| Oleth-7 | 5,70 |
| Kaliumoleinseife, 12,5%ig in Wasser | 12,75 |
| Kaliumricinusseife, 12,5%ig in Wasser | 3,45 |
| Plantaren^{®} 2000 | 0,53 |
| Titandioxid Anatas, Typ AS 05 | 0,48 |
| Cetiol^{®} V | 3,45 |
| Cetylalkohol | 16,80 |
| Glycerinmonostearat NSE | 2,85 |
| Phospholipid PTC | 0,85 |
| Tetrasodium EDTA | 0,46 |
| Kieselsäure, hochdispers, pyrogen | 0,22 |
| p-Toluylendiamin | 1,33 |
| Resorcin | 0,48 |
| 3-Aminophenol | 0,10 |
| 1-Methoxy-2-amino-4-(2-hydroxyethylamino)-benzol | 0,02 |
| 1,2-Propylenglykol USP | 1,05 |
| Methoxybutanol | 1,43 |
| Ammoniak, 25%ig in Wasser | ad pH 9,0 |
| Ascorbinsäure | 0,06 |
| Mirapol^{®} A15 | 0,19 |
| Parfüm | 0,43 |
| Wasser | ad 100,00 |

Dieses Mittel wurde mit einer wäßrigen, 6%igen Wasserstoffperoxidlösung im Verhältnis 1:2 vermischt und auf eine dunkelblonde Normalhaarsträhne aufgetragen. Nach einer Einwirkzeit von 30min bei 25°C wurde die Strähne mit Wasser ausgespült, nachshampooniert und mit dem Fön getrocknet.
Die so erhaltene Strähne war hellbraun eingefärbt.

## Patentansprüche

1. Mittel zur pflegenden Behandlung keratinischer Fasern, insbesondere menschlicher Haare, **dadurch gekennzeichnet, daß** es mindestens ein Tensid der Formel (I) enthält 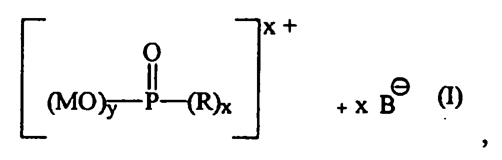 in der y für eine ganze Zahl von 0 bis 2 steht, x für eine ganze Zahl von 1 bis 3 steht mit der Maßgabe, daß x + y = 3 ist,
M für Wasserstoff, ein Äquivalent eines Alkali- oder Erdalkalimetallkations, ein Ammoniumkation oder einen Alkylrest mit 1 bis 4 Kohlenstoffatomen steht, der gegebenenfalls mit einer oder mehreren Hydroxygruppen substituiert ist,
B für ein Äquivalent eines physiologisch verträglichen Anions steht und
R für einen Rest der Formel (II) steht, 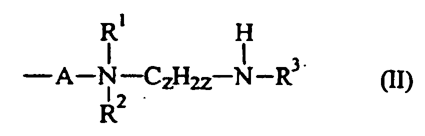 in der z für eine ganze Zahl von 1 bis 4 steht,
R¹ und R² unabhängig voneinander für einen C₁- bis C₄-Alkylrest stehen, der gegebenenfalls mit einer oder mehreren Hydroxygruppe(n) oder einer Acylgruppe substituiert ist,
A für -O-CH₂-CH₂-CH₂-, -O-CH₂-CH₂- oder -O-CH₂-CHOH-CH₂- steht und
R³ steht für
(a) einen verzweigten oder unverzweigten, gesättigten C₈- bis C₁₈-Acylrest oder
(b) einen verzweigten oder unverzweigten, einfach oder mehrfach ungesättigten C₈- bis C₁₈-Acylrest,
sowie mindestens eine konditionierende Komponente und
mindestens einen Farbstoff und/oder mindestens ein Farbstoffvorprodukt.

2. Mittel nach Anspruch 1, **dadurch gekennzeichnet, daß** es mindestens ein anionisches Tensid, insbesondere eine Seife, enthält.

3. Mittel nach einem der Ansprüche 1 bis 2, **dadurch gekennzeichnet, daß** die konditionierende Komponente eine niedermolekulare quaternäre Ammoniumverbindung ist.

4. Mittel nach einem der Anspüche 1 bis 3, **dadurch gekennzeichnet, daß** die konditionierende Komponente ein kationisches Polymeres ist.

5. Mittel nach Anspruch 4, **dadurch gekennzeichnet, daß** die konditionierende Komponente ein quaternisiertes Cellulosederivat ist.

6. Mittel nach einem der Ansprüche 4 oder 5, **dadurch gekennzeichnet, daß** die konditionierende Komponente Polyquaternium-2 ist.

7. Mittel nach einem der Anspüche 1 bis 6, **dadurch gekennzeichnet, daß** die konditionierende Komponente ein quaternisiertes Eiweißhydrolysat ist.

8. Mittel nach einem der Ansprüche 1 bis 7, **dadurch gekennzeichnet, daß** die konditionierende Komponente ein Silikonöl ist.

9. Mittel nach einem der Ansprüche 1 bis 8, **dadurch gekennzeichnet, daß** es mindestens ein Farbstoffvorprodukt vom Typ der Entwickler enthält.

10. Mittel nach einem der Ansprüche 1 bis 9, **dadurch gekennzeichnet**, es als Farbstoffvorprodukt mindestens eine Indol- und/oder Indolinderivat enthält.

11. Mittel nach einem der Ansprüche 1 bis 10, **dadurch gekennzeichnet, daß** es mindestens einen direktziehenden Farbstoff und/oder einen natürlichen Farbstoff enthält.

12. Verwendung eines der Mittel der Ansprüche 1 bis 11 zum Färben keratinischer Fasern.

## Claims

1. An agent for the care treatment of keratin fibers, in particular human hair, **characterized in that** it comprises at least one tenside of the formula (I) 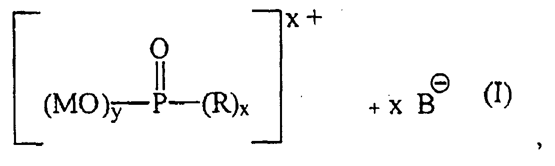 in which y is an integer from 0 to 2, x is an integer from 1 to 3 with the proviso that x + y = 3,
M is hydrogen, an equivalent of an alkali metal or alkaline earth metal cation, an ammonium cation or an alkyl radical having 1 to 4 carbon atoms, which is optionally substituted by one or more hydroxyl groups,
B is an equivalent of a physiologically compatible anion and
R is a radical of the formula (II), 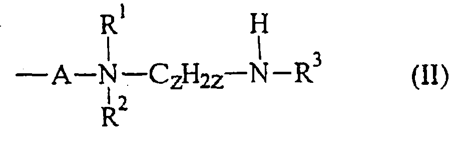 in which z is an integer from 1 to 4,
R¹ and R², independently of one another, are a C₁₋C₄-alkyl radical, which is optionally substituted by one or more hydroxyl group(s) or an acyl group,
A is -O-CH₂-CH₂-CH₂-, -O-CH₂-CH₂- or -O-CH₂-CHOH-CH₂- and
R³ is
(a) a branched or unbranched, saturated C₈-C₁₈-acyl radical or
(b) a branched or unbranched, mono- or polyunsaturated C₈-C₁₈-acyl radical
and at least one conditioning component, and
at least one dye and/or at least one dye precursor.

2. The agent as claimed in claim 1, **characterized in that** it comprises at least one anionic tenside, in particular a soap.

3. The agent as claimed in either claim 1 or 2, **characterized in that** the conditioning component is a low molecular weight quaternary ammonium compound.

4. The agent as claimed in any of claims 1 to 3, **characterized in that** the conditioning component is a cationic polymer.

5. The agent as claimed in claim 4, **characterized in that** the conditioning component is a quaternized cellulose derivative.

6. The agent as claimed in either claim 4 or 5, **characterized in that** the conditioning component is polyquaternium-2.

7. The agent as claimed in any of claims 1 to 6, **characterized in that** the conditioning component is a quaternized protein hydrolyzate.

8. The agent as claimed in any of claims 1 to 7, **characterized in that** the conditioning component is a silicone oil.

9. The agent as claimed in any of claims 1 to 8, **characterized in that** it comprises at least one dye precursor of the developer type.

10. The agent as claimed in any of claims 1 to 9, **characterized in** it comprises at least one indole and/or indolene derivative as dye precursor.

11. The agent as claimed in any of claims 1 to 10, **characterized in that** it comprises at least one substantive dye and/or a natural dye.

12. The use of one of the agents of claims 1 to 11 for coloring keratin fibers.

## Revendications

1. Agent pour le soin de fibres kératiniques, en particulier de cheveux humains, **caractérisé** est ce qu'il contient au moins un agent tensioactif de formule (I) 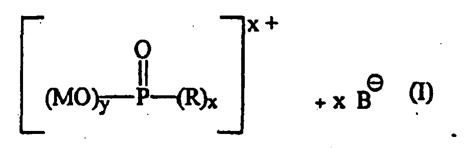 dans laquelle y représente un nombre entier de 0 à 2, x représente un nombre entier de 1 à 3, à condition que x + y = 3,
M représente hydrogène, un équivalent d'un cation de métal alcalin ou alcalino-terreux, un cation d'ammonium ou un radical alkyle comprenant 1 à 4 atomes de carbone, qui est le cas échéant substitué par un ou plusieurs groupes hydroxy,
B représente un équivalent d'un anion physiologiquement acceptable et
R représente un radical de formule (II), 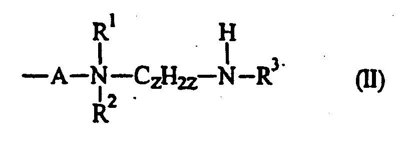 dans laquelle z vaut un nombre entier de 1 à 4,
R¹ et R² représentent, indépendamment l'un de l'autre, un radical alkyle en C₁ à C₄, qui est le cas échéant substitué par un ou plusieurs groupes hydroxy ou un groupe acyle,
A représenté -O-CH₂-CH₂-CH₂-, -O-CH₂-CH₂- ou -O-CH₂-CHOH-CH₂- et R³ représente
(a) un radical acyle en C₈ à C₁₈ ramifié ou non ramifié, saturé ou
(b) un radical acyle en C₈ à C₁₈ ramifié ou non ramifié, monoinsaturé ou polyinsaturé,
ainsi qu'au moins un composant de conditionnement et
au moins un colorant et/ou au moins un précurseur d'un colorant.

2. Agent selon la revendication 1, **caractérisé en ce qu'**il contient au moins un agent tensioactif anionique, en particulier un savon.

3. Agent selon l'une quelconque des revendications 1 à 2, **caractérisé en ce que** le composant de conditionnement est un composé d'ammonium quaternaire de bas poids moléculaire.

4. Agent selon l'une quelconque des revendications 1 à 3, **caractérisé en ce que** le composant de conditionnement est un polymère cationique.

5. Agent selon la revendication 4, **caractérisé en ce que** le composant de conditionnement est un dérivé de cellulose quaternisé.

6. Agent selon l'une quelconque des revendications 4 ou 5, **caractérisé en ce que** le composant de conditionnement est le Polyquaternium-2.

7. Agent selon l'une quelconque des revendications 1 à 6, **caractérisé en ce que** le composant de conditionnement est un hydrolysat de protéines quaternisées.

8. Agent selon l'une quelconque des revendications 1 à 7, **caractérisé en ce que** le composant de conditionnement est une huile de silicone.

9. Agent selon l'une quelconque des revendications 1 à 8, **caractérisé en ce qu'**il contient au moins un précurseur d'un colorant du type agent de développement.

10. Agent selon l'une quelconque des revendications 1 à 9, **caractérisé en ce qu'**il contient comme précurseur de colorant au moins un dérivé d'indole et/ou d'indoline.

11. Agent selon l'une quelconque des revendications 1 à 10, **caractérisé en ce qu'**il contient au moins un colorant montant directement sur la fibre et/ou un colorant naturel.

12. Utilisation d'un agent selon les revendications 1 à 11 pour teindre des fibres kératiniques.
